# EUROPEAN PATENT SPECIFICATION

(11) **EP 3 427 719 B1**
(45) Date of publication and mention of the grant of the patent: **06.03.2024**
(21) Application number: 17181363.7
(22) Date of filing: 14.07.2017
(51) Int. Cl.: A61K 8/20, A61Q 5/08, A61Q 5/10, A61K 8/22, A61K 8/19, A61K 8/23

(54) **AQUEOUS BLEACHING AND DYEING COMPOSITION FOR KERATIN FIBERS, PROCESS, KIT AND USE THEREOF**
WÄSSRIGE BLEICH- UND FÄRBEZUSAMMENSETZUNG FÜR KERATINFASERN, VERFAHREN, KIT UND VERWENDUNG DAVON
COMPOSITION AQUEUSE DE DÉCOLORATION OU TEINTURE DES FIBRES KÉRATINIQUES, PROCÉDÉ, KIT ET UTILISATION DE CELUI-CI

(43) Date of publication of application: 16.01.2019
(73) Proprietor: Kao Germany GmbH, 64297 Darmstadt (DE)
(72) Inventor: NÖCKER, Bernd, 64297 Darmstadt (DE); Meulbrouck, Celine, 64297 Darmstadt (DE)
(74) Representative: Grit, Mustafa

(56) References cited:
- EP-A1- 1 714 677
- EP-A1- 2 417 959
- WO-A1-99/13840
- JP-A- H09 249 538
- JP-A- 2002 068 976
- US-A- 3 811 833
- US-A- 5 951 969
- US-A1- 2006 137 111
- US-A1- 2010 015 805
- US-A1- 2014 230 163

## Description

The present invention relates to a bleaching and/or dyeing composition for keratin fibers, preferably human keratin fibers, more preferably human hair. Furthermore, a process, kit-of-parts, and use of the composition are disclosed.

Ammonia-based hair bleaching and/or dyeing compositions and processes are commonly known to consumers with the disadvantage of experiencing a noticeable and disliked ammonia smell. Moreover, despite the strong typical olfactory experience, the bleaching and/or coloring result remains in some cases unsatisfactory because ammonia evaporates over process time and consequently the pH of the hair color shifts to a less effective value. Additionally, ammonia-based bleaching and/or dyeing compositions are known to confer a certain degree of damage to keratin fibers. Hence, mildness to the hair and its perception of the customer is an important factor for such compositions. Apart from consumer experience, there are technical reasons which render ammonia incompatible with certain hair color compounds, such as direct hair dyes or oxidation sensitive compounds. However, as ammonia is such an effective compound for bleaching and/or dyeing, its presence is often required to achieve the desired effect.

In EP2476405 and EP2931227 reduction of ammonia smell was addressed wherein either ammonia was replaced by an organic amine or a specific emulsion was formulated to reduce ammonia evaporation. However, both solutions are not satisfactory because organic amine salts commonly deliver a weaker performance than ammonia and a high amount of fatty compounds may lead to highly viscous compositions which lack good applicability on hair.

In EP2062565 ammonia and/or its salts are either added to anhydrous compositions or are present in the alkaline dyeing composition. EP2468240 discloses oxidizing compositions comprising ammonium carbonate and/or ammonium bicarbonate at pH ranges above 6.8. EP2598435 discloses an oxidizing composition comprising an ammonium salt of a polymer, copolymers and/or crosspolymers comprising acryloyl dimethyltaurate monomers at a pH of around 4. As this disclosure is unsuitable to deliver the effect of the present invention, it is not to be seen as forming part of the present invention. Therefore, compositions of the present invention are free of copolymers and/or crosspolymers comprising acryloyl dimethyltaurate monomers.

JP 2002 068976 A discloses an oxidizing composition comprising 0,4% by weight of monoammonium phosphate.

In summary of the prior art, none of the documents disclose a satisfactory solution to reducing ammonia smell while maintaining the performance similar or better than ammonia-based products. After extensive research, inventors of the present invention have found that the presence of certain ammonia salts in the oxidizing composition solves the problems of above.

Therefore, the first object of the present invention is an aqueous oxidizing composition according to claim 1 for bleaching and/or dyeing of keratin fibers, preferably human keratin fibers, more preferably human hair, characterized in that it comprises hydrogen peroxide, one or more non-polymeric salt(s) of NH₄⁺, and it has a pH in the range of 1 to 6.

The second object of the present invention is a process for bleaching and/or dyeing of keratin fibers, preferably human keratin fibers, more preferably human hair, characterized in that it comprises the following steps:
a) mixing a composition as defined above with a second composition comprising an alkalizing agent to form a ready-to-use composition having a pH in the range from 7 to 12,
b) applying the ready-to-use composition onto keratin fibers and leaving it for a time period of 1 min to 45 min,
c) rinsing-off the composition from keratin fibers,
d) optionally shampooing the keratin fibers,
e) optionally drying the keratin fibers.

The third object of the present invention is kit-of-parts comprising in a first container the composition as defined above, and at least one second separate container comprising a composition with an alkalizing agent.

Further object of the present invention is the use of the composition as defined above on keratin fibers, preferably human keratin fibers, more preferably human hair, for lightening, bleaching and/or dyeing.

The one or more non-polymeric salt(s) of NH₄⁺ according to the present invention is/are selected from ammonium acetate, ammonium benzoate, ammonium carbamate, ammonium chloride, ammonium ferric pentetate, ammonium fluoride, ammonium iodide, ammonium bromide, ammonium lactate, ammonium nitrate, ammonium phosphate, diammonium phosphate, triammonium phosphate, ammonium propionate, ammonium sulphate, ammonium sulphite, ammonium thiocyanate, ammonium xylenesulfonate, ammonium citrate, diammonium citrate, triammonium citrate. The preferred non-polymeric salt(s) of NH₄⁺ is/are selected from ammonium sulphate, ammonium chloride, ammonium acetate, and mono-, di- and/or triammonium citrate.

The most preferred non-polymeric salt(s) of NH₄⁺ is ammonium sulphate.

The total concentration of the non-polymeric salt(s) of NH₄⁺ is in the range of 0.5% to 10% by weight, preferably in the range of 0.5% to 8% by weight, and more preferably in the range of 1 % to 6% by weight, calculated to the total of the composition.

The oxidizing agent according to the present invention is hydrogen peroxide which is present at a concentration in the range of 0.5% to 20% by weight, more preferably in the range of 1 % to 9% by weight, calculated to the total of the composition.

The composition of the present invention has a pH in the range of 1 to 6, preferably 1.5 to 5, more preferably in the range of 1.8 to 4. In principle, the pH of the composition can be adjusted with any organic and/or inorganic acid(s) or base or their mixtures. Suitable acids are phosphoric acid, hydrochloric acid as the inorganic ones and to the organic acids the well-known citric acid and lactic acid, glycolic acid, glyoxylic acid, hydroxyacrylic acid, glyceric acid, malic acid and tartaric acid and of the dicarboxylic acids are malonic acid, succinic acid, glutaric acid, adipic acid, maleic acid, fumaric acid and phtalic acid. Suitable bases are sodium hydroxide or potassium hydroxide. The most preferred acid is phosphoric acid.

The composition may comprise organic alkyl and/or alkanol amine salt according to the general structure 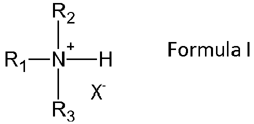 wherein R1, R2, and R3 are independently selected from H, linear C1-C6 alkyl which may be substituted with one hydroxyl group, or branched C3-C12 alkyl or alkanol, wherein at least one of R1, R2, or R3 is different from H, and X- is an anion.

The organic amine according to the structure above may be selected from mono- and/or diethanolamine, butyl ethanolamine, butyl diethanolamine, dibutyl ethanolamine, methylethanolamine, triethanolamine, N-lauryl diethanolamine, diisopropanolamine, dimethyl isopropanolamine, isopropanolamine, triisopropanolamine, isobutanolamine. The counterion X- is selected from chloride and/or hydrogen chloride, nitrate, sulphate, phosphate, hydrogenphosphate, dihydrogenphosphate, citrate, acetate, sulphite, benzoate, salicylate. The most preferred organic amine salt is mono- or diethanolamine hydrochloride. The concentration of organic amine salts is preferably in the range of 0.01% to 2% by weight, more preferably 0.1% to 1.5% by weight, calculated to the total of the composition.

The composition is in the form of an emulsion. For formation of emulsion, the composition comprises lipophilic compounds selected from natural and/or vegetable oils, petrolatum-based compounds, linear or branched, saturated or unsaturated fatty alcohols with C12 to C22, and fatty acid esters consisting of linear or branched, saturated or unsaturated fatty acids with C12 to C22 being esterified with linear or branched primary alcohols with C3 to C12, and silicones.

Suitable natural and/or vegetable oils are olive oil, almond oil, avocado oil, wheatgerm oil, and castor oil.

Suitable petrolatum-based compounds are liquid paraffins, especially paraffinum perliquidum and paraffinum subliquidum, and mineral oil, in particular white mineral oil.

Suitable comprises fatty compounds selected from linear or branched, saturated or unsaturated fatty alcohols with C12 to C22 are lauryl alcohol, tridecyl alcohol, myristyl alcohol, pentadecyl alcohol, cetyl alcohol, palmitoleyl alcohol, heptadecyl alcohol, stearyl alcohol, nonadecyl alcohol, arachidyl alcohol, behenyl alcohol, and/or their mixtures.

Suitable examples for fatty acid esters consisting of linear or branched, saturated or unsaturated fatty acids with C12 to C22 being esterified with linear or branched primary alcohols with C3 to C18 are octyl palmitate, isocetyl palmitate, isopropyl palmitate, octyl stearate, oleyl oleate, and myristyl myristate, as well as their mixtures.

The composition also comprises lipophilic ingredients such as silicones for example linear polysiloxanes such as dimethicones with various consistency and dimethiconols, aminated silicones with primary, secondary, tertiary or quaternary ammonium groups such as amodimethicone, polysilicone 9, and quaternium 80, cyclic silicones such as cyclomethicones, arylated silicones such as phenyl trimethicone; C10- to C36-fatty acid triglycerides, as well as their mixtures.

Total concentration of these lipophilic compounds is in the range of 0.1% to 20% by weight, preferably from 1% to 15% by weight, and more preferably from 2% to 10% by weight, calculated to the total of the composition.

The composition further comprises one or more surfactants selected from cationic surfactants and/or non-ionic surfactants and/or anionic surfactants and/or zwitterionic/amphoteric surfactants, preferably non-ionic surfactants.

Suitable non-ionic surfactants are in general all commonly known non-ionic surfactants available on the market.

Suitable nonionic surfactants are alkyl polyglycosides according to the general structure:

R²³O(R²⁴O)ₜZₓ

Wherein Z denotes a reducing carbohydrate with C₅ to C₆, R²³ is an alkyl group with C₈ to C₁₈, R²⁴ is ethyl or propyl, t ranges from 0 to 10, and x ranges from 1 to 5. Suitable compounds according to this structure are C₉-C₁₁ alkylpolyglycoside, the structures disclosed in EP-A 70 074, and JP 2015-123019A.

The preferred compound according to the structure of above is decyl glucoside.

Suitable examples for non-ionic surfactants are fatty alcohol ethoxylates of the following general structure

R¹⁸ (OCH₂CH₂)ₙ₄ OH

wherein R¹⁸ is straight or branched, saturated or unsaturated alkyl chain which may be synthetic or natural with a C chain length in the range of 8 to 40, preferably 9 to 30 and more preferably 9 to 24 and n4 is a number in the range of 5 to 40, preferably 9 to 30.

Non-limiting suitable examples of the fatty alcohol ethoxylates are C9-11 Pareth-6, C9-11 Pareth-8, C9-15 Pareth-8, C11-13 Pareth-9, C11-13 Pareth-10, C11-15 Pareth-5, C11-15 Pareth-7, C11-15 Pareth-9, C11-15 Pareth-12, C11-15 Pareth-15, C11-15 Pareth-20, C11-15 Pareth-30, C11-15 Pareth-40, C11-21 Pareth-10, C12-13 Pareth-5, C12-13 Pareth-6, C12-13 Pareth-7, C12-13 Pareth-9, C12-13 Pareth-10, C12-13 Pareth-15, C12-13 Pareth-23, C12-14 Pareth-5, C12-14 Pareth-7, C12-14 Pareth-9, C12-14 Pareth-11, C12-14 Pareth-12, C12-15 Pareth-5, C12-15 Pareth-7, C12-15 Pareth-9, C12-15 Pareth-10, C12-15 Pareth-11, C12-15 Pareth-12, C12-16 Pareth-5, C12-16 Pareth-7, C12-16 Pareth-9, C13-15 Pareth-21, C14-15 Pareth-7, C14-15 Pareth-8, C14-15 Pareth-11, C14-15 Pareth-12, C14-15 Pareth-13, C20-22 Pareth-30, C20-40 Pareth-10, C20-40 Pareth-24, C20-40 Pareth-40, C20-40 Pareth-95, C22-24 Pareth-33, Beheneth-5, Beheneth-10, Beheneth-15, Beheneth-20, Beheneth-25, Beheneth-30, Ceteareth-5, Ceteareth-6, Ceteareth-7, Ceteareth-10, Ceteareth-11, Ceteareth-12, Ceteareth-15, Ceteareth-20, Ceteareth-25, Ceteareth-30, Ceteareth-35, Ceteareth-40, Laureth-5, Laureth-10, Laureth-15, Laureth-20, Laureth-25, Laureth-30, Laureth-40, Myreth-5, Myreth-10, Ceteth-5, Ceteth-10, Ceteth-15, Ceteth-20, Ceteth-25, Ceteth-30, Ceteth-40, Oleth-5, Oleth-10, Oleth-15, Oleth-20, Oleth-25, Oleth-30, Oleth-40, Steareth-5, Steareth-10, Steareth-15, Steareth-20, Steareth-25, Steareth-30, Steareth-35, and Steareth-40. They may also be comprised in the compositions as a mixture of more than one surfactant.

Further suitable nonionic surfactants are polypropylene glycol ethers of fatty alcohol according to general structure

R¹⁹ (OCH₂ (CH₃) CH₂)ₙ₅ OH

wherein R¹⁹ is straight or branched, saturated or unsaturated fatty alcohol which may be synthetic or natural with a C chain length in the range of 8 to 40, preferably 9 to 30 and more preferably 9 to 24 and n5 is a number in the range of 1 to 40, preferably 3 to 30.

Suitable non-limiting examples are PPG-3 Caprylyl ether, PPG-5 Caprylyl ether, PPG-10 Caprylyl ether, PPG-10 Cetyl ether, PPG-20 Cetyl ether, PPG-28 Cetyl ether, PPG-30 Cetyl ether, PPG-7 Lauryl ether, PPG-10 Lauryl ether, PPG-10 Oleyl ether, PPG-20 Oleyl ether, PPG-23 Oleyl ether, PPG-30 Oleyl ether, PPG-11 Stearyl ether and PPG-15 Stearyl ether.

Further suitable nonionic surfactants are polyethylene glycol fatty acid esters of the following general structure

R²⁰ C(O) (OCH₂CH₂)ₙ₆ OH

wherein R²⁰ is straight or branched, saturated or unsaturated alkyl group which may be synthetic or natural with a C chain length in the range of 7 to 39, preferably 9 to 29 and more preferably 9 to 23 and n6 is a number in the range of 5 to 40, preferably 9 to 30.

Suitable non-limiting examples are PEG-8 Behenate, PEG-8 Caprate, PEG-8 Caprylate, PEG-5 Cocoate, PEG-8 Cocoate, PEG-9 Cocoate, PEG-10 Cocoate, PEG-15 Cocoate, PEG-6 Isopalmitate, PEG-6 Isostearate, PEG-8 Isostearate, PEG-9 Isostearate, PEG-10 Isostearate, PEG-12 Isostearate, PEG-20 Isostearate, PEG-30 Isostearate, PEG-40 Isostearate, PEG-6 Laurate, PEG-8 Laurate, PEG-9 Laurate, PEG-10 Laurate, PEG-12 Laurate, PEG-14 Laurate, PEG-20 Laurate, PEG-30 Laurate, PEG-8 Myristate, PEG-20 Myristate, PEG-5 Oleate, PEG-6 Oleate, PEG-7 Oleate, PEG-8 Oleate, PEG-9 Oleate, PEG-10 Oleate, PEG-11 Oleate, PEG-12 Oleate, PEG-15 Oleate, PEG-20 Oleate, PEG-30 Oleate, PEG-32 Oleate, PEG-6 Palmitate, PEG-18 Palmitate, PEG-20 Palmitate, PEG-5 Stearate, PEG-6 Stearate, PEG-7 Stearate, PEG-8 Stearate, PEG-9 Stearate, PEG-10 Stearate, PEG-12 Stearate, PEG-14 Stearate, PEG-15 Stearate, PEG-20 Stearate, PEG-25 Stearate, PEG-30 Stearate, PEG-35 Stearate and PEG-40 Stearate.

Further suitable nonionic surfactants are polypropylene glycol fatty acid esters of the following general structure

R²¹ C(O) (OCH₂(CH₃) CH₂)ₙ₈ OH

wherein R²¹ is straight or branched, saturated or unsaturated alkyl group which may be synthetic or natural with a C chain length in the range of 7 to 39, preferably 9 to 29 and more preferably 9 to 23 and n8 is a number in the range of 1 to 40, preferably 9 to 30.

Suitable non-limiting examples are PPG-15 Isostearate, PPG-9 Laurate, PPG-26 Oleate and PPG-36 Oleate.

Further suitable nonionic surfactants are polyethylene glycol and polypropylene glycol ether of fatty alcohols of the following general structure

R²² (OCH₂ (CH₃) CH₂)ₙ₉ (OCH₂CH₂)ₙ₁₀ OH

wherein R²² is straight or branched, saturated or unsaturated alkyl group which may be synthetic or natural with a C chain length in the range of 7 to 39, preferably 9 to 29 and more preferably 9 to 23 and n9 and n10 may be the same or different and are a number in the range of 1 to 40.

Further suitable nonionic surfactants are ethoxylated triglycerides. Well known and commonly used examples are ethoxylated castor oil such as PEG-40 hydrogenated castor oil or and PEG-60 hydrogenated castor oil.

Preferably the non-ionic surfactant(s) are selected from linear or branched, saturated or unsaturated fatty alcohols with C6 to C22 being modified with 3-30 repeating units of ethylene oxide and/or propylene oxide.

Suitable anionic surfactants are selected from compounds according to the structure 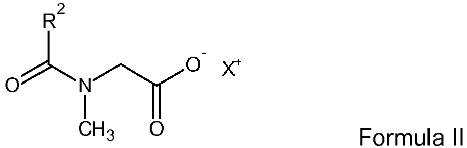 wherein R² is a straight or branched, substituted or unsubstituted, saturated or unsaturated alkyl chain with a carbon number of C₉ to C₂₁, preferably R² is a straight alkyl chain with a carbon number of C₉ to C₁₇, and X⁺ is a cation selected from sodium, potassium, magnesium and ammonium ions, which are compounds based on the amino acid sarcosin which are commonly known as sarcosinates.

Suitable compounds are, for example, cocoyl sarcosinate and its salts, lauroyl sarcosinate and its salts, myristoyl sarcosinate and its salts, stearoyl sarcosinate and its salts, oleoyl sarcosinate and its salts, palmitoyl sarcosinate and its salts. Salts are formed with cations selected from sodium, potassium, magnesium, and ammonium ions.

The preferred surfactant according to the structure of formula II is sodium lauroyl sarcosinate.

Suitable anionic surfactants are alkyl sulfate or preferably ethoxylated alkyl ether sulfate surfactant or mixtures thereof with an alkyl chain length of C₁₀ to C₂₂.

Suitable surfactants are laureth sulfates, coceth sulfate, pareth sulfate, capryleth sulphate, myreth sulfate, oleth sulfate, deceth sulfate, trideceth sulfate, coco sulphate, C₁₀-C₁₆ alkyl sulphate, C₁₁-C₁₅ alkyl sulphate, C₁₂-C₁₈ alkyl sulphate, C₁₂-C₁₅ alkyl sulphate, C₁₂-C₁₆ alkyl sulphate, C₁₂-C₁₃ alkyl sulfate, lauryl sulphate, myristyl sulphate, palm kernel sulphate, cetearyl sulfate, cetyl sulphate, decyl sulphate, oleyl sulphate, behenyl sulphate and/or their salts. All of the aforementioned anionic surfactants may or may not be ethoxylated at various degrees.

Cations for the surfactants may be selected from sodium, potassium, magnesium and/or ammonium.

Suitable anionic surfactant is sodium laureth sulfate with 1-5 ethylene oxide units.

The composition may comprise the anionic surfactants at a total concentration in the range of 0.1% to 10% by weight, preferably in the range of 0.5% to 8% by weight, more preferably in the range of 1% to 5% by weight, calculated to the total of the composition.

The amphoteric surfactant may be selected from compounds according to the general structure(s) III and/or IV 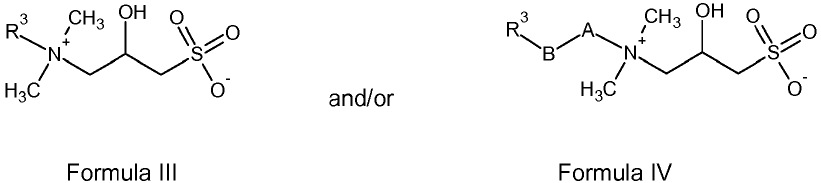 wherein R³ is a straight or branched, saturated or unsaturated, substituted or unsubstituted alkyl chain with a carbon number of C₁₀ to C₂₂, preferably R³ is a straight alkyl chain with a carbon number of C₁₀ to C₁₆, A is a straight alkyl chain with a carbon number of C₁ to C₆ or a branched alkyl chain with a carbon number of C₃ to C₆, preferably A is a linear alkyl chain with a carbon number of C₃, and B is an amide or an ester group.

Suitable compounds are known as hydroxysultain surfactants, such as cocoamidopropyl hydroxysultaine, laurylamidopropyl hydroxysultaine, erucamidopropyl hydroxysultaine, lauryl hydroxysultaine, and cocoyl hydrodroxysultaine.

Other suitable zwitterionic surfactants are known as betaines. A suitable example is cocoamidopropylbetaine

The preferred amphoteric/zwitterionic surfactant is/are lauryl hydroxysultaine and cocoamidopropylbetaine.

The composition may further comprise cationic surfactants of quaternary ammonium structure according to

R₅ R₆ R₇ R₈ N

wherein R₅ is an alky chain having C length of 8 to 30 which may be saturated or unsaturated, straight or branched, R₆ is an alkyl chain having C length of 1 to 30 which may be saturated or unsaturated, straight or branched, R₅ and R₆ additionally may take the structures of

   R₉ C(O) O (CH₂)ₙ₁ or R₁₀ C(O) NH (CH₂)ₙ₁
wherein R₉ is an alkyl chain with a C length of 7 to 29 which may be saturated or unsaturated, straight or branched and n1 is a number between 1 and 4,
R₇ and R₈ are same or the different alkyl chain with a C length of 1 to 4 which may be straight or branched (only for C₃ and C₄), wherein all alkyl chains may comprise one or more substituents such as hydroxyl- or (poly)-ethoxy groups.

Anions for the cationic surfactants may be selected from chloride, sulfate, or nitrate.

The total concentration of surfactants may be in the range of 0.1% to 10% by weight, preferably 0.5% to 7.5% by weight, more preferably 1% to 5% by weight, calculated to the total of the composition.

The composition of the present invention further comprises one or more thickeners. In a preferred embodiment of the present invention, the composition comprises one or more thickening polymers selected from anionic, nonionic, cationic and amphoteric polymers, preferably selected from polymers resulting in a solution and/or dispersion with a viscosity of at least 500 mPa•s measured at a polymer concentration of 1% by weight in water and at 20°C with a Brookfield viscometer, such as at 10 rpm for 1 min, with an appropriate spindle.

Suitable polymers are cellulose polymers, alginates, polysaccharides and acrylic acid polymers, preferably methyl cellulose, ethyl cellulose, hydroxyethylcellulose, methylhydroxyethylcellulose, methylhydroxypropylcellulose, carboxymethyl cellulose, alginic acids, sodium alginates, ammonium alginates, calcium alginates, gum arabic, guar gum or xanthan gum, dehydroxanthan gum or acrylic acid polymers known with the CTFA adopted name Carbomer and its derivatives.

The preferred polymers are dehydroxanthan gum, xanthan gum, and polymeric anionic thickeners, namely Carbomer and its derivatives.

Of particular advantageous use are thickeners which are commonly known as associative thickeners. Preferred are copolymers and/or crosspolymers which comprise an acrylate and/or methacrylate monomer unit and at least one more hydrophobic unit such as alkyl chains. Examples are acrylates/c10-30 alkyl acrylate crosspolymer, acrylates/steareth-20 methacrylate copolymer, acrylates/stearyl acrylate/dimethicone methacrylate copolymer, acrylates/beheneth-25 methacrylate copolymer, acrylates/lauryl acrylate/stearyl acrylate/ ethylamine oxide methacrylate copolymer.

The composition preferably comprises thickening agents at a total concentration in the range of 0.1% to 5%, preferably, 0.2% to 3%, more preferably 0.25% to 2.5% and most preferably 0.3% to 2% by weight calculated to the total of the composition.

The composition of the present invention further comprises hair direct dyes selected from non-ionic, cationic and/or anionic hair direct dyes.

Suitable anionic direct dyes are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium. Among those, the most preferred anionic dyestuffs are Acid Red 52, DC Violet 2, DC Red 33, DC Orange 4, DC Red 27, DC Yellow 10, HC Blue 18, HC Red 18, and HC Yellow 16.

Suitable cationic dyes are in principle those available on the market for cosmetic hair colouring applications. For this purpose, special reference is made to the PCT application WO 95/15144 of Ciba-Geigy AG. Some examples to those are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14, Basic Yellow 57, Basic red 51, Basic Yellow 87, HC Blue 17 and Basic Orange 31. The most preferred ones are Basic red 51, Basic Yellow 87 and Basic Orange 31 sold by BASF, HC Blue 17, Basic Blue 124.

Suitable neutral dyes including nitro dyes are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

The composition may comprise one or more hair direct dye at a total concentration of 0.001% to 10% by weight, preferably 0.005% to 7.5% by weight, and more preferably 0.01% to 5% by weight, calculated to the total of the composition. The composition can also comprise a mixture of several direct dyes, i.e., an anionic, a cationic and/or nonionic ones. In such a case the dyes may be mixed at any ratio with each other.

The composition further comprises stabilizing agents. Suitable stabilizing agents may be selected from 8-hydroxy-quinoline sulfate monohydrate, salicylic acid, and 4-hydroxyacetanilid. Suitable concentration of stabilizers is in the range of 0.01% to 0.5% by weight, preferably 0.02% to 0.1% by weight, calculated to the total of the composition.

The composition of the present invention is mixed with a second composition comprising an alkalizing agent. The second composition is a powder composition or a liquid composition. Although being a powder composition, it may comprise liquid compounds at a low amount. For liquid compositions, the composition may be a solution or emulsion wherein emulsion form is preferred.

The second composition comprises non-ammonia alkalizing agents such as 2-amino-2-methylpropanol, sodium metasilicate, and organic alkyl and/or alkanol amine salt(s) according to the general structure 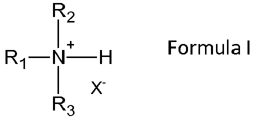 wherein R1, R2, and R3 are independently selected from H, linear C1-C6 alkyl which may be substituted with one hydroxyl group, or branched C3-C12 alkyl or alkanol, wherein at least one of R1, R2, or R3 is different from H, and X- is an anion.

The organic amine according to the structure above may be selected from mono- and/or diethanolamine, butyl ethanolamine, butyl diethanolamine, dibutyl ethanolamine, methylethanolamine, triethanolamine, N-lauryl diethanolamine, diisopropanolamine, dimethyl isopropanolamine, isopropanolamine, triisopropanolamine, isobutanolamine. The counterion X- is selected from chloride and/or hydrogen chloride, nitrate, sulphate, phosphate, hydrogenphosphate, dihydrogenphosphate, citrate, acetate, sulphite, benzoate, salicylate. The most preferred organic amine salt is mono- or diethanolamine hydrochloride.

The concentration of alkalizing agents in the second composition is preferably in the range of 1% to 20% by weight, more preferably 2% to 15% by weight, calculated to the total of the composition.

In one embodiment of the present invention, the second composition comprises non-polymeric salts of NH₄⁺ at a total concentration below 3% by weight, calculated to the total of the second composition.

It is a preferred embodiment of the present invention that the second composition is free of non-polymeric salts of NH₄⁺.

The second composition further comprises oxidative hair dyes.

Suitable non-limiting examples of oxidative dye precursor classes are p-phenylendiamines, p-aminophenols, and heterocyclic compounds such as diaminopyrazols and substituted pyrimidines, and suitable coupling substances are resorcinols, m-aminophenols, m-phenylendiamines, pyridines and its derivatives, and naphthols.

Non-limiting examples of the oxidative dye precursor compounds are p-phenylenediamine, p-aminophenol, 2,5-diamino-toluene, 2-n-propyl or 2-ethyl-p-phenylenediamine, 2,6-di-methyl-p-phenylene- diamine, 2-(2,5-diaminophenyl) ethanol, 1-amino-4-bis-(2'-hydroxy-ethyl)amino- benzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-β-hydroxypropyl aminobenzene, 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 3,5-diamino-1,2,4-triazole, 4-aminophenol and the derivatives thereof such as 4-amino-3-methylphenol, 2-chloro-4-aminophenol, 2,6-dichloro-4-aminophenol, 2,4-diamino-phenol, 2,6-dibromo-4-aminophenol, tetraamino pyrimidines, triaminohydroxy pyrimidines, diaminomono- and -dihydroxy pyrimidines, aminotriazines, 5-amino salicylic acid and/or 1,2,4-triamino benzene, 2,5-diaminopyridine, 2,3-diaminopyridine, 2,6-diaminopyridine, 3-amino-2-methyl amino-6-methoxypyridine, 2-dimethyl-5-aminopyridine, 2-dimethyl aminoethyl-3-hydroxypyridine, 2-amino-4,6-dimethyl pyridine, 2-amino-3-hydroxypyridine, 3-amino-2(β-hydroxyethyl amino)-6-methoxy pyridine, 2,6-dimethyl amino-5-aminopyridine, 2-di(hydroxyethyl) amino-5-aminopyridine, 2-hydroxyethyl amino-5-aminopyridine, 4-hydroxy-2,5,6-triaminopyrimidine and/or the water-soluble salts thereof, and mixture thereof.

The total concentration of the dye precursors (developing substances) customarily ranges between 0.001 to 5%, preferably 0.01 to 4% and more preferably 0.05 to 3%, and most preferably 0.1 to 2% by weight, calculated to the total of the second composition.

Suitable non-limiting examples of the coupling substance if present in the second composition are 5-amino-2-methylphenol, 2-methyl-5-hydroxyethylaminophenol, 2,4,-diaminophenoxyethanol, 2-amino-4-hydroxyethylaminoanisol, 2-methyl-5-amino-6-chlorphenol, 1,3-bis(2,4-diaminophenoxy)propane, 2-bis(2-hydroxyethyl)aminotoluene, 2-amino-5-methylphenol, resorcinol, 2-methyl-resorcinol, 4-chlororesorcinol, 2-amino-4-chlorophenol, 5-amino-4-methoxy-2-methylphenol, 2-aminophenol, 3-amino-phenol, 1-methyl-2-hydroxy-4-aminobenzene, 3-N,N-dimethyl aminophenol, 2,6-dihydroxy-3,5-dimethoxypyridine, 5-amino-3-methylphenol, 6-amino-3-methylphenol, 1,3-diamino- benzene, 1-amino-3-(2'-hy-droxyethylamino)benzene, 1-amino-3-[bis(2'-hydroxy-ethyl) amino]benzene, α-naphthol, 4,6-dichlororesorcinol, 1,3-diamino-toluene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1,2-methyldioxy benzene, 2,4-diamino-3-chlorophenol, 5-amino-2-methoxyphenol and/or 1-methoxy-2-amino-4-(2'-hydroxyethyl amino)benzene or the water-soluble salts thereof and mixture thereof.

Coupling substance(s) in the second composition as reaction partners for the developing substance(s) are present in approximately the same molecular proportions as the developing substances, i.e. at a total concentration in the range of 0.001 to 5%, preferably 0.01 to 4% and more preferably 0.05 to 3%, and most preferably 0.1 to 2% by weight, calculated to the total of the second composition.

The second composition comprises hair direct dyes. Suitable hair direct dyes are identical to the ones disclosed for the composition according to the present invention. Prior to application onto hair, the composition of the present invention must be mixed with a second composition to yield a ready-to-use composition with a pH in the range of 7 to 12. Preferred mixing ratios by weight of the composition according to the present invention to second composition are 1:1 to 2:1, but other mixing ratios are possible as well.

The following examples are to illustrate the invention, but not to limit it.

### EXAMPLES

### EXAMPLE 1

The following oxidizing compositions were prepared by conventional formulation methods:

| **Ingredient** | **Inventive composition A** | **Inventive composition B** | **Comparative composition** |
|---|---|---|---|
| Cetearyl alcohol | 5.0 | 5.0 | 5.0 |
| Ceteareth-30 | 2.5 | 2.5 | 2.5 |
| Ammonium chloride | 5.0 | - | - |
| Ammonium sulfate | - | 5.0 | - |
| Hydrogen peroxide | 12.0 | | |
| Phosphoric acid | q.s. pH 4.0 | | |
| Water | Ad 100.0 | | |

The following powder compositions were prepared:

| **Ingredient** | **Powder composition A** | **Powder composition B** |
|---|---|---|
| Paraffin oil | 10.0 | 10.0 |
| Corn starch | 7.5 | 7.5 |
| Sodium bicarbonate | 2.0 | 2.0 |
| 2-Amino-2-methylpropanol | 0.5 | 0.5 |
| Sodium metasilicate | 2.0 | 2.0 |
| Ammonium sulphate | - | 5.0 |
| Diatomaceous earth | Ad 100.0 | |

The following mixtures were prepared:

| **Ready-to-use compositions** | **Aqueous oxidizing composition** | **Powder composition** | **Mixture pH** |
|---|---|---|---|
| 1 | 2 parts inventive composition A | 1 part powder composition A | 8.30 |
| 2 | 2 parts inventive composition B | 1 part powder composition A | 8.11 |
| 3 | 2 part comparative composition | 1 part powder composition B | 8.91 |

2 g of the resulting ready-to-use mixtures were applied onto human hair streaks (Caucasian, 21 cm long) purchased from Fischbach + Miller, Laupheim, Germany.

The compositions were left onto hair for 30 min at room temperature. Then the compositions were rinsed-off with water and the hair streaks were shampooed. Hair streaks were then blow-dried.

Lightening results were measured prior to bleaching and after bleaching by spectrophotometrical analysis with a Datacolor 45G CT instrument delivered from Datacolor Inc., Lawrenceville, NJ, USA. The difference in lightening was calculated as △L.

The following results were obtained:

| **Ready-to-use compositions** | Δ**L** |
|---|---|
| 1 | 23.02 |
| 2 | 22.69 |
| 3 | 20.47 |

As a result, the inventive processes delivered higher lightening in comparison to the comparative compositions. The difference in lightening exceeded 2 ΔL units, and, therefore, was perceivable by the naked human eye.

The following examples are within the scope of the invention.

### EXAMPLE 2

| | **% by weight** |
|---|---|
| Stearyl alcohol | 7.5 |
| Isopropyl palmitate | 5.0 |
| PEG-40 hydrogenated castor oil | 3.0 |
| 8-hydroxy-quinoline sulfate monohydrate | 0.03 |
| Ammonium sulfate | 3.0 |
| Ammonium chloride | 1.0 |
| Monoethanolamine hydrochloride | 0.5 |
| Hydrogen peroxide | 9.0 |
| Phosphoric acid | q.s. ad pH 2.0 |
| Water | ad 100.0 |

The composition is employed upon mixture with either powder composition from example 1 in the same ratio.

### EXAMPLE 3

| | **% by weight** |
|---|---|
| Behenyl alcohol | 5.0 |
| Sunflower oil | 10.0 |
| Steareth-20 | 2.0 |
| Sodium lauryl sulfate | 1.0 |
| Salicylic acid | 0.02 |
| 4-hydroxyacetanilid | 0.02 |
| Ammonium sulfate | 6.0 |
| Monoethanolamine hydrochloride | 1.5 |
| HC Blue 18 | 0.2 |
| Dehydroxanthan gum | 0.5 |
| Hydrogen peroxide | 6.0 |
| Phosphoric acid | q.s. ad pH 2.0 |
| Water | ad 100.0 |

The composition is employed upon mixture with either powder composition from example 1 in the same ratio.

### EXAMPLE 4

| | **% by weight** |
|---|---|
| Stearyl alcohol | 8.0 |
| Pareth-20 | 2.0 |
| Salicylic acid | 0.02 |
| 4-hydroxyacetanilid | 0.02 |
| Ammonium chloride | 3.0 |
| Monoethanolamine hydrochloride | 2.0 |
| HC Red 18 | 0.2 |
| Acrylates copolymer (acrylates/steareth-20 methacrylate copolymer) | 0.5 |
| Hydrogen peroxide | 9.0 |
| Phosphoric acid | q.s. ad pH 4.5 |
| Water | ad 100.0 |

The composition is employed upon mixture with either powder composition from example 1 in the same ratio.

### EXAMPLE 5

### Oxidative composition

| | **% by weight** |
|---|---|
| Stearyl alcohol | 8.0 |
| Pareth-20 | 2.0 |
| Salicylic acid | 0.02 |
| 4-hydroxyacetanilid | 0.02 |
| Ammonium chloride | 3.0 |
| Monoethanolamine hydrochloride | 2.0 |
| HC Red 18 | 0.2 |
| Acrylates copolymer (acrylates/steareth-20 methacrylate copolymer) | 0.5 |
| Hydrogen peroxide | 9.0 |
| Phosphoric acid | q.s. ad pH 4.5 |
| Water | ad 100.0 |

### Dyeing composition

| | **% by weight** |
|---|---|
| Cetearyl alcohol | 10.0 |
| Cocamide MEA | 4.0 |
| Sodium lauryl sulphate | 1.5 |
| Propylene glycol | 2.0 |
| Cetyltrimonium chloride | 0.5 |
| 2,5,6-Triamino-4-hydroxypyrimidine sulfate | 0.01 |
| 2,5-Diaminotoluene sulfate | 0.55 |
| 4-Chlororesorcinol | 0.17 |
| Resorcinol | 0.05 |
| 3-Aminophenol | 0.03 |
| Sodium sulfite | 1.0 |
| Aminomethyl propanol | 2.0 |
| Monoethanolamine | q.s. to pH 10.0 |
| Fragrance, preservative | q.s. |
| Water | to 100 |

The oxidative composition and dyeing composition were mixed in a weight ratio 1:1.

## Claims

1. An aqueous oxidizing composition for bleaching and/or dyeing of keratin fibers, preferably human keratin fibers, more preferably human hair, **characterized in that** it comprises hydrogen peroxide, one or more non-polymeric salt(s) of NH₄⁺, and it has a pH in the range of 1 to 6,
wherein one or more non-polymeric salt(s) of NH₄⁺ is/are selected from ammonium acetate, ammonium benzoate, ammonium carbamate, ammonium chloride, ammonium ferric pentetate, ammonium fluoride, ammonium iodide, ammonium bromide, ammonium lactate, ammonium nitrate, ammonium phosphate, diammonium phosphate, triammonium phosphate, ammonium propionate, ammonium sulphate, ammonium sulphite, ammonium thiocyanate, ammonium xylenesulfonate, ammonium citrate, diammonium citrate, triammonium citrate,
wherein the total concentration of the non-polymeric salt(s) of NH₄⁺ is in the range of 0.5% to 10% by weight, calculated to the total of the composition,
and wherein it is an emulsion.

2. The composition according to claims 1 and/or 2 **characterized in that** the one or more non-polymeric salt(s) of NH₄⁺ is/are selected from ammonium sulfate, ammonium chloride, ammonium acetate, and mono-, di- and/or triammonium citrate.

3. The composition according to any of the preceding claims **characterized in that** the non-polymeric salt of NH₄⁺ is ammonium sulfate.

4. The composition according to any of the preceding claims **characterized in that** it comprises organic alkyl and/or alkanol amine salt according to the general structure 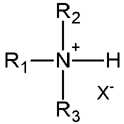 wherein R1, R2, and R3 are independently selected from H, linear C1-C6 alkyl which may be substituted with one hydroxyl group, or branched C3-C12 alkyl or alkanol, wherein at least one of R1, R2, or R3 is different from H, and X- is an anion.

5. The composition according to any of the preceding claims **characterized in that** the total concentration of the non-polymeric salt(s) of NH₄⁺ is in the range of 0.5% to 8% by weight, and more preferably in the range of 1% to 6% by weight, calculated to the total of the composition.

6. The composition according to any of the preceding claims **characterized in that** the concentration of hydrogen peroxide is in the range of 0.5% to 20% by weight, more preferably in the range of 1% to 9% by weight, calculated to the total of the composition.

7. The composition according to any of the preceding claims **characterized in that** it comprises lipophilic compounds selected from natural and/or vegetable oils, petrolatum-based compounds, linear or branched, saturated or unsaturated fatty alcohols with C12 to C22, and fatty acid esters consisting of linear or branched, saturated or unsaturated fatty acids with C12 to C22 being esterified with linear or branched primary alcohols with C3 to C18, and silicones.

8. The composition according to any of the preceding claims **characterized in that** it comprises one or more surfactants selected from cationic surfactants and/or nonionic surfactants and/or anionic surfactants surfactants and/or zwitterionic/amphoteric surfactants, preferably non-ionic surfactants.

9. The composition according to any of the preceding claims **characterized in that** the pH of the composition is in the range 1.5 to 5, more preferably in the range of 1.8 to 4.

10. The composition according to any of the preceding claims **characterized in that** it comprises one or more thickeners.

11. A process for bleaching and/or dyeing of keratin fibers, preferably human keratin fibers, more preferably human hair, **characterized in that** it comprises the following steps:
a) mixing a composition according to any of the claims 1 to 10 with a second composition comprising an alkalizing agent to form a ready-to-use composition having a pH in the range from 7 to 12,
b) applying the ready-to-use composition onto keratin fibers and leaving it for a time period of 1 min to 45 min,
c) rinsing-off the composition from keratin fibers,
d) optionally shampooing the keratin fibers,
e) optionally drying the keratin fibers.

12. Kit-of-parts comprising in a first container the composition according to any of the claims 1 to 10, and at least one second separate container comprising a composition with an alkalizing agent.

13. Use of the composition according to the claims 1 to 10 on keratin fibers, preferably human keratin fibers, more preferably human hair, for lightening, bleaching and/or dyeing.

## Patentansprüche

1. Wässrige oxidierende Zusammensetzung zum Bleichen und/oder Färben von Keratinfasern, vorzugsweise menschlichen Keratinfasern, besonders bevorzugt menschlichem Haar, **dadurch gekennzeichnet, dass** sie Wasserstoffperoxid, ein oder mehrere nichtpolymere(s) Salz(e) von NH₄⁺ enthält und einen pH-Wert im Bereich von 1 bis 6 aufweist,
wobei ein oder mehrere nichtpolymere(s) Salz(e) von NH₄⁺ ausgewählt ist/sind aus Ammoniumacetat, Ammoniumbenzoat, Ammoniumcarbamat, Ammoniumchlorid, Ammoniumeisenpentetat, Ammoniumfluorid, Ammoniumiodid, Ammoniumbromid, Ammoniumlactat, Ammoniumnitrat, Ammoniumphosphat, Diammoniumphosphat, Triammoniumphosphat, Ammoniumpropionat, Ammoniumsulfat, Ammoniumsulfit, Ammoniumthiocyanat, Ammoniumxylensulfonat, Ammoniumzitrat, Diammoniumzitrat, Triammoniumzitrat,
wobei die Gesamtkonzentration des nichtpolymeren Salzes/der nichtpolymeren Salze von NH₄⁺ im Bereich von 0,5 bis 10 Gew.-% liegt, berechnet auf die Gesamtzusammensetzung,
und bei dem es sich um eine Emulsion handelt.

2. Zusammensetzung nach Anspruch 1 und/oder 2, **dadurch gekennzeichnet, dass** das (die) eine(n) nichtpolymere(n) Salz(e) von NH₄⁺ ausgewählt ist (sind) aus Ammoniumsulfat, Ammoniumchlorid, Ammoniumacetat und Mono-, Di- und/oder Triammoniumcitrat.

3. Zusammensetzung nach einem der vorhergehenden Ansprüche, **dadurch gekennzeichnet, dass** das nichtpolymere Salz von NH₄⁺ Ammoniumsulfat ist.

4. Zusammensetzung nach einem der vorangehenden Ansprüche, **dadurch gekennzeichnet, daß** sie ein organisches Alkyl- und/oder Alkanolaminsalz der allgemeinen Struktur 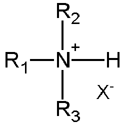 worin R1, R2 und R3 unabhängig voneinander ausgewählt sind aus H, linearem C1-C6-Alkyl, das mit einer Hydroxylgruppe substituiert sein kann, oder verzweigtem C3-C12-Alkyl oder -Alkanol, wobei mindestens einer der Reste R1, R2 oder R3 von H verschieden ist, und X- ein Anion ist.

5. Zusammensetzung nach einem der vorhergehenden Ansprüche, **dadurch gekennzeichnet, dass** die Gesamtkonzentration des (der) nichtpolymeren Salzes (Salze) von NH₄⁺ im Bereich von 0,5 bis 8 Gew.-% und vorzugsweise im Bereich von 1 bis 6 Gew.-%, bezogen auf die Gesamtmenge der Zusammensetzung, liegt.

6. Zusammensetzung nach einem der vorhergehenden Ansprüche, **dadurch gekennzeichnet, daß** die Konzentration an Wasserstoffperoxid im Bereich von 0,5 bis 20 Gew.-%, vorzugsweise im Bereich von 1 bis 9 Gew.-%, bezogen auf die gesamte Zusammensetzung, liegt.

7. Zusammensetzung nach einem der vorhergehenden Ansprüche, **dadurch gekennzeichnet, dass** sie lipophile Verbindungen enthält, die ausgewählt sind aus natürlichen und/oder pflanzlichen Ölen, Verbindungen auf Petrolatumbasis, linearen oder verzweigten, gesättigten oder ungesättigten Fettalkoholen mit C12 bis C22 und Fettsäureestern, die aus linearen oder verzweigten, gesättigten oder ungesättigten Fettsäuren mit C12 bis C22 bestehen, die mit linearen oder verzweigten primären Alkoholen mit C3 bis C18 verestert sind, und Silikonen.

8. Zusammensetzung nach einem der vorhergehenden Ansprüche, **dadurch gekennzeichnet, dass** sie ein oder mehrere Tenside, ausgewählt aus kationischen Tensiden und/oder nichtionischen Tensiden und/oder anionischen Tensiden und/oder zwitterionischen/amphoteren Tensiden, vorzugsweise nichtionische Tenside, enthält.

9. Zusammensetzung nach einem der vorhergehenden Ansprüche, **dadurch gekennzeichnet, dass** der pH-Wert der Zusammensetzung im Bereich von 1,5 bis 5, vorzugsweise im Bereich von 1,8 bis 4 liegt.

10. Die Zusammensetzung nach einem der vorhergehenden Ansprüche, **dadurch gekennzeichnet, dass** sie ein oder mehrere Verdickungsmittel enthält.

11. Verfahren zum Bleichen und/oder Färben von Keratinfasern, vorzugsweise menschlichen Keratinfasern, besonders bevorzugt menschlichen Haaren, **dadurch gekennzeichnet, dass** es die folgenden Schritte umfasst:
a) Mischen einer Zusammensetzung nach einem der Ansprüche 1 bis 10 mit einer zweiten Zusammensetzung, die ein Alkalisierungsmittel enthält, um eine gebrauchsfertige Zusammensetzung mit einem pH-Wert im Bereich von 7 bis 12 zu bilden,
b) Auftragen der gebrauchsfertigen Zusammensetzung auf die Keratinfasern und Einwirkenlassen für eine Zeitspanne von 1 min bis 45 min,
c) Abspülen der Zusammensetzung von den Keratinfasern,
d) optionales Shampoonieren der Keratinfasern,
e) optionales Trocknen der Keratinfasern.

12. Teilesatz, enthaltend in einem ersten Behälter die Zusammensetzung nach einem der Ansprüche 1 bis 10 und mindestens einen zweiten separaten Behälter, der eine Zusammensetzung mit einem Alkalisierungsmittel enthält.

13. Verwendung der Zusammensetzung gemäß den Ansprüchen 1 bis 10 auf Keratinfasern, vorzugsweise menschlichen Keratinfasern, besonders bevorzugt menschlichem Haar, zum Aufhellen, Bleichen und/oder Färben.

## Revendications

1. Composition aqueuse oxydante pour la décoloration et/ou la teinture des fibres kératiniques, de préférence des fibres kératiniques humaines, plus préférentiellement des cheveux humains, **caractérisée par le fait qu'**elle comprend du peroxyde d'hydrogène, un ou plusieurs sel(s) non polymérique(s) de NH₄⁺, et qu'elle a un pH compris entre 1 et 6,
dans lequel un ou plusieurs sel(s) non polymérique(s) de NH₄⁺ est/sont choisi(s) parmi l'acétate d'ammonium, le benzoate d'ammonium, le carbamate d'ammonium, le chlorure d'ammonium, le pentétate ferrique d'ammonium, le fluorure d'ammonium, l'iodure d'ammonium, le bromure d'ammonium, le lactate d'ammonium, nitrate d'ammonium, phosphate d'ammonium, phosphate diammonique, phosphate triammonique, propionate d'ammonium, sulfate d'ammonium, sulfite d'ammonium, thiocyanate d'ammonium, xylène sulfonate d'ammonium, citrate d'ammonium, citrate diammonique, citrate triammonique,
dans lequel la concentration totale du (des) sel(s) non polymérique(s) de NH₄⁺ est comprise entre 0,5% et 10% en poids, calculée par rapport à la composition totale, et dans lequel il s'agit d'une émulsion.

2. Composition selon les revendications 1 et/ou 2, **caractérisée par le fait que** le ou les sel(s) non polymérique(s) de NH₄⁺ est/sont choisi(s) parmi le sulfate d'ammonium, le chlorure d'ammonium, l'acétate d'ammonium et le citrate de mono-, di- et/ou triammonium.

3. Composition selon l'une quelconque des revendications précédentes, **caractérisée par le fait que** le sel non polymérique de NH₄⁺ est le sulfate d'ammonium.

4. Composition selon l'une quelconque des revendications précédentes **caractérisée en ce qu'**elle comprend un sel organique d'alkyl et/ou d'alcanolamine selon la structure générale suivante 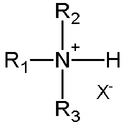 où R₁, R₂ et R₃ sont indépendamment choisis parmi H, alkyle linéaire en C₁-C₆ pouvant être substitué par un groupe hydroxyle, ou alkyle ramifié en C₃-C₁₂ ou alcanol, où au moins un des R₁, R₂ ou R₃ est différent de H, et X⁻ est un anion.

5. La composition selon l'une des revendications précédentes est **caractérisée par le fait que** la concentration totale du (des) sel(s) non polymérique(s) de NH₄⁺ est comprise entre 0.5% et 8% en poids, et de préférence entre 1% et 6% en poids, calculée par rapport au total de la composition.

6. La composition selon l'une des revendications précédentes est **caractérisée par le fait que** la concentration de peroxyde d'hydrogène est comprise entre 0.5% et 20% en poids, de préférence entre 1% et 9% en poids, par rapport au total de la composition.

7. La composition selon l'une quelconque des revendications précédentes **caractérisée en ce qu'**elle comprend des composés lipophiles choisis parmi les huiles naturelles et/ou végétales, les composés à base de pétrolatum, les alcools gras linéaires ou ramifiés, saturés ou insaturés en C₁₂ à C₂₂, et les esters d'acides gras constitués d'acides gras linéaires ou ramifiés, saturés ou insaturés en C₁₂ à C₂₂ estérifiés par des alcools primaires linéaires ou ramifiés en C₃ à C₁₈, et les silicones.

8. La composition selon l'une quelconque des revendications précédentes est **caractérisée par le fait qu'**elle comprend un ou plusieurs agents tensioactifs choisis parmi les agents tensioactifs cationiques et/ou les agents tensioactifs non ioniques et/ou les agents tensioactifs anioniques et/ou les agents tensioactifs zwitterioniques/amphotériques, de préférence les agents tensioactifs non ioniques.

9. La composition selon l'une des revendications précédentes est **caractérisée par le fait que** le pH de la composition est compris entre 1.5 et 5, plus préférentiellement entre 1.8 et 4.

10. La composition selon l'une des revendications précédentes **caractérisée par le fait qu'**elle comprend un ou plusieurs épaississants.

11. Procédé de décoloration et/ou de teinture de fibres kératiniques, de préférence de fibres kératiniques humaines, plus particulièrement de cheveux humains, **caractérisé en ce qu'**il comprend les étapes suivantes :
a) mélanger une composition selon l'une des revendications 1 à 10 avec une seconde composition comprenant un agent alcalinisant pour former une composition prête à l'emploi ayant un pH compris entre 7 et 12,
b) appliquer la composition prête à l'emploi sur les fibres kératiniques et la laisser agir pendant une période de 1 à 45 minutes,
c) rincer la composition sur les fibres kératiniques,
d) éventuellement, shampouiner les fibres kératiniques,
e) séchage éventuel des fibres kératiniques.

12. Kit de pièces comprenant dans un premier récipient la composition selon l'une des revendications 1 à 10, et au moins un second récipient séparé comprenant une composition avec un agent alcalinisant.

13. Utilisation de la composition selon les revendications 1 à 10 sur les fibres kératiniques, de préférence les fibres kératiniques humaines, plus préférentiellement les cheveux humains, pour l'éclaircissement, la décoloration et/ou la teinture.
